Europäisches Patentamt

European Patent Office

Office européen des brevets

(11) Publication number: **0 368 330**
**A1**

## (12) EUROPEAN PATENT APPLICATION

(21) Application number: **89120865.4**

(22) Date of filing: **10.11.89**

(51) Int. Cl.⁵: **A61M 25/01**

(30) Priority: **11.11.88 JP 283930/88**

(43) Date of publication of application:
**16.05.90 Bulletin 90/20**

(84) Designated Contracting States:
**BE DE FR GB IT NL SE**

(71) Applicant: **Terumo Kabushiki Kaisha**
**No. 44-1, Hatagaya 2-chome Shibuya-ku**
**Tokyo 151(JP)**

(72) Inventor: **Kazuhito, Ishihara c/o Terumo K. K.**
**150, Maimaigi-cho Fujinomiya-shi**
**Shizuoka-ken(JP)**

(74) Representative: **Casalonga, Axel et al**
**BUREAU D.A. CASALONGA - JOSSE**
**Morassistrasse 8**
**D-8000 München 5(DE)**

(54) Guide wire assembly for medical instrument.

(57) A guide wire assembly for guiding a medical instrument wherein a hollow connector (10) capable of connecting to a catheter hub and to a syringe for the liquid medicine injection is mounted at the proximal end of the guide wire (11). The guide wire (11) has an outer diameter smaller than the inner diameter of the connector (10) and is fixed to the inner wall of the connector (10) thereby forming a fluid passageway between the guide wire (11) and the inner wall of the connector (10), allowing a fluid to pass therethrough. A valve (12) may be mounted in the connector (10) for closing the fluid passageway.

EP 0 368 330 A1

F I G. 4

## Guide wire assembly for medical instrument

The present invention relates to a guide wire assembly for guiding a catheter into a blood vessel for the treatment or inspection of a diseased portion within the blood vessel.

An intravascular catheter is used mainly for injecting a liquid medicine or a contrast media into a diseased portion within the blood vessel for the treatment or inspection of the diseased portion. A conventional catheter of this type consists of a flexible tube formed of a synthetic resin and a hub mounted to the proximal end of the flexible tube. For introducing the catheter into a diseased portion within a blood vessel, a guide wire is introduced in advance into the blood vessel for guiding the catheter to reach the diseased portion within the blood vessel. After arrival of the catheter at the diseased portion, the guide wire is withdrawn, followed by injecting a contrast medium, medicine, embolism resolvent, etc. from the proximal end of the catheter into the diseased portion within the blood vessel.

As described above, it is necessary to withdraw the guide wire from the catheter in the prior art before injection of liquid medicine or the like from the proximal end of the catheter, making the operation troublesome and time-consuming.

Further, where a guide wire and a catheter are inserted together into a blood vessel, it is necessary to select a path of insertion at a branched portion of the blood vessel. For the selection, the connector (hub) mounted at the proximal end of the catheter is rotated in general for twisting the guide wire. In the prior art, however, the torque of the connector is unlikely to be transmitted without fail to the guide wire. Also, the bonding strength between the guide wire and the connector is insufficient for achieving a satisfactory transmission of force.

An object of the present invention is to provide a guide wire assembly for guiding a medical instrument constructed such that the guide wire once introduced into a blood vessel or the like need not be withdrawn even when a liquid medicine or the like is injected into the blood vessel or even when a fluid such as humor or gas is withdrawn from the body.

Another object is to provide a guide wire assembly for medical instrument constructed such that the guide wire is strongly bonded to a connector to ensure a sufficient transmission of the torque of the connector to the guide wire.

To achieve these objects, a connector capable of connection to a catheter hub and to a syringe for the liquid medicine injection is mounted at the proximal end of the guide wire.

According to the present invention, there is provided a guide wire assembly for medical instrument, comprising a hollow connector open at both ends and capable of connection at the rear end to another medical instrument, and a guide wire having an outer diameter substantially smaller than the inner diameter at the hollow portion of the connector, the proximal end portion of said wire being fixed to the inner wall of the connector, with the distal portion projecting through the front end of the connector, wherein a fluid is capable of passing from the rear end to the front end of the connector.

A conventional guide wire, e.g., a super-elastic metal wire, can be used in the present invention. It is possible to coat the metal wire with a synthetic resin and, further, with a water-soluble high molecular weight material. It is also possible to coat the outer surface of the catheter with a water-soluble high molecular weight material so as to reduce the frictional resistance between the catheter and the inner wall of a blood vessel. Further, it is possible to mix an X-ray impermeable substance into a resin coating layer on the catheter or on the super-elastic metal wire. In this case, the operation for introducing the catheter can be performed while observing an image formed by X-ray.

The "super-elastic wire" used herein denotes an alloy wire which brings about an apparent plastic deformation of several % to about 10% upon receipt of a stress under temperatures higher than the temperature at which a reverse transformation of the alloy is completed and which is completely brought back to the original state upon removal of the stress. A Ti-Ni alloy consisting essentially of 50.3 to 52.0 atomic %, most preferably 50.5 to 51.0 atomic %, of Ni and the balance substantially of Ti can be used for forming the super-elastic wire. It is also possible to use a Ti-Ni-X alloy, "X" representing at least one kind of a third element.

The present invention also provides a guide wire assembly for medical instrument, which further comprises a valve mechanism, e.g., a two-way valve, mounted in a part of the connector for closing the fluid passageway, when desired, so as to prevent leakage of blood or the like from the catheter. In the connector used in the present invention, a male type luer taper and a female type luer taper may be formed in the front and rear end portions, respectively.

In the present invention, a connector provided with a male type luer taper and a female type luer taper is mounted to the proximal end of a guide wire. Also, the hub of a catheter and a syringe are connected to the connector of the particular construction. It follows that the guide wire need not be withdrawn from the catheter, which is once introduced into a blood vessel, during the injection of a liquid medicine, a contrast media or the like into the blood vessel, or during the withdrawal of fluid such as humor or gas from the body. In other words, the injection can be performed, with the guide wire kept inserted into the blood vessel. It should also be noted that the proximal end of the wire is simply embedded in the inner wall of the hollow connector. As a result, a thick fluid passageway can be secured within the hollow connector. In addition, the proximal end of the guide wire can be strongly fixed to the connector, with the result that the force of torque of the connector is sufficiently transmitted to the guide wire.

This invention can be more fully understood from the following detailed description when taken in conjunction with the accompanying drawings, in which:

Fig. 1 is a cross sectional view showing the gist portion of a guide wire assembly for medical instrument according to one embodiment of the present invention;

Fig. 2 is a cross sectional view along line II-II shown in Fig. 1;

Fig. 3 is a cross sectional view along line III-III shown in Fig. 1;

Fig. 4 is a cross sectional view showing a guide wire assembly for medical instrument according to another embodiment of the present invention;

Fig. 5 is a cross sectional view along line V-V shown in Fig. 4; and

Fig. 6 schematically shows how to use a guide wire assembly of the present invention.

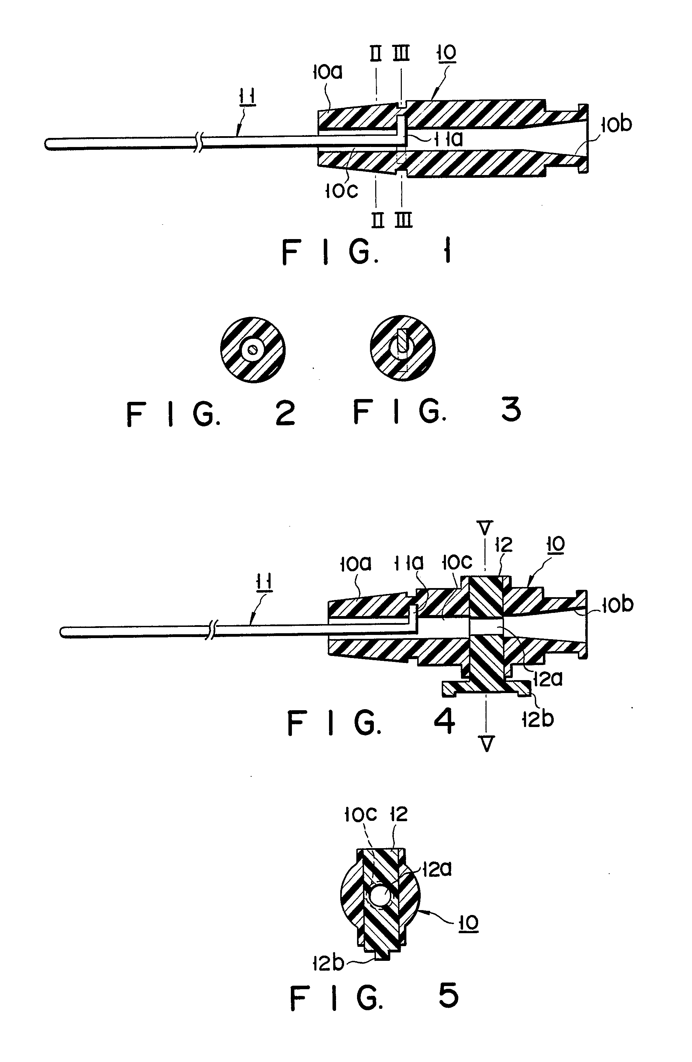

As seen from Figs. 1 to 3 collectively showing one embodiment of the present invention, a guide wire assembly of the present invention comprises a hollow connector 10 open at both ends and a guide wire 11 having a proximal end 11a embedded in the inner wall of the hollow connector 10. A male type luer taper 10a is formed at the front end portion of the connector 10. Also, a female type luer taper 10b is formed at the rear end portion of the connector 10. The connector 10 is formed of a synthetic resin such as nylon.

The guide wire 11 is formed of a super-elastic Ni-Ti binary alloy wire containing 50.3 atomic % or less of Ni, and has an outer diameter substantially smaller than the inner diameter of a hollow portion 10C of the connector 10. For example, the outer diameter of the wire 11 is 0.6 mm and the inner diameter of the hollow portion 10c of the connector is 2 mm. As seen from Fig. 3, the proximal end 11a of the wire 10 is bent at nearly the right angles and is embedded in the inner wall of the hollow portion of the connector 10. The straight portion of the guide wire 10 extends along the axis of the hollow portion of the connector 10 as seen from Fig. 2 and projects outwards through the male type luer taper 10a. A catheter is engaged with the male type luer taper 10a. The length of the guide wire 11 is determined in conjunction with the length of the catheter. In general, 15 to 50 mm of the distal end portion of the guide wire 11 projects from the distal end of the catheter. Alternatively, more than 50 mm of the distal end portion of the guide wire may project from the distal end of the catheter, or the distal end of the guide wire may be positioned within the catheter.

For bonding the guide wire 11 to the connector 10, it is desirable to employ an insert molding in which the bent proximal end 11a of the guide wire 11 is inserted in advance in a mold for molding the connector 10. Alternatively, the portions of the male type luer taper 10a and the female type luer taper 10b are separately molded in advance, and the bent proximal end 11a of the guide wire 11 is held between these portions.

In the embodiment described above, the proximal end 11a, which is bent at nearly the right angles, is embedded in the inner wall of the connector 10 for joining the guide wire 11 to the connector 10. However, an optional method can be employed for joining the guide wire 11 to the connector 10 as far as the fluid passageway formed by the hollow portion of the connector 10 is not markedly narrowed. For example, the proximal end of the guide wire 11 may be bent in advance in substantially T-shape and the tips of the bent portion may be embedded in the inner wall of the hollow portion of the connector 10, as denoted by imaginary lines in Figs. 1 and 3.

The guide wire 11 may be coated with an urethane resin by a method similar to that disclosed in, example, Unexamined Published Japanese Patent Application No. 61-45775 so as to modify the surface. The urethane resin layer may be further coated with a water-soluble high molecular material such as polyvinylpyrrolidone, sodium methylvinylether maleic anhydride, polyacrylic amide hydrolyzate, sodium alginate, sodium polyvinylsulfonate, ammonium salt of methylvinylether maleic anhydride and a quarternary compound of polyacrylamide. This coating is wetted by water or an aqueous solution so as to impart lubricity to the guide wire 11.

The distal end portion of the guide wire 11 may be somewhat bent to conform with the shape of the blood vessel into which the guide wire 11 is to be inserted. Further, an X-ray impermeable material may be mixed into the guide wire 11 through the resin coating layers. In this case, a catheter may be smoothly introduced into the blood vessel by utilizing an X-ray image-forming apparatus.

Figs. 4 and 5 collectively show another embodiment of the present invention. This embodiment is substantially equal to the embodiment shown in Figs. 1 to 3, except that, in this embodiment, a two-way valve 12 capable of closing the liquid passageway, when desired, is rotatably provided in the intermediate portion of the connector 10 so as to prevent leakage of blood from the catheter connected to the male type luer taper 10a. Of course, the reference numerals common with Figs. 1 to 5 denote the same members of the assembly. The two-way valve 12 is nearly of a columnar shape. As seen from the drawings, a through-hole 12a substantially conforming in cross section with the hollow portion 10c of the connector 10 is formed in the central portion of the two-way valve 12. The valve 12 also comprises a knob 12b projecting from one end. The liquid passageway formed by the hollow portion 10c of the connector 10 is opened or closed by moving the knob 12b to rotate the valve 12.

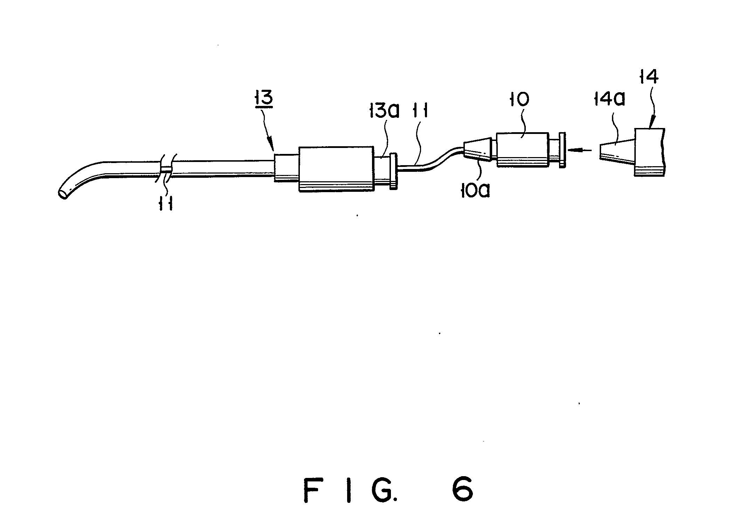

Fig. 6 schematically shows how to use the guide wire assembly of the present invention. In the first step, the guide wire assembly is inserted through a hub 13a of a catheter 13 so as to allow the male type luer taper 10a of the connector 10 to be engaged with a female type luer taper formed within the hub 13a of the catheter. Under this condition, the guide wire 11 and the catheter 13 are introduced into a desired portion within, for example, a blood vessel while properly controlling the projecting length of the guide wire 11. The guide wire is rotated when arriving at a branched portion of the blood vessel to permit the distal end of the catheter 13 to be guided to the desired portion. Then, a male type luer taper portion 14a of a syringe 14 is inserted into the connector 10 of the guide wire assembly so as to be engaged with the female type luer taper 10b of the connector 10. A liquid medicine, an image-forming agent or the like, which is injected through the syringe 14, passes through the hollow portion 10C of the connector 10 so as to be guided to the desired portion within the blood vessel through the catheter 13. When desired, the two-way valve 12 is rotated so as to close the fluid passageway formed within the connector 10 and, thus, to prevent the leakage of blood from the catheter. In the case of using a three-way valve in place of the two-way valve 12, it is possible to inject simultaneously a plurality of liquid medicine, etc. from a plurality of supply ports.

end to another medical instrument, and a guide wire (11) having an outer diameter substantially smaller than the inner diameter at the hollow portion of the connector (10), the proximal end portion of said wire (11) being fixed to the inner wall of the connector (10), with the distal portion projecting through the front end of the connector (10), characterized in that a fluid is capable of passing from the rear end to the front end of the connector (10).

2. The guide wire assembly according to claim 1, characterized in that a valve mechanism (12) capable of selectively closing the liquid passageway is mounted within the connector (10) rearward of the fixing portion of the proximal end of the guide wire (11) to the connector (10).

3. The guide wire assembly according to claim 1, characterized in that a male type luer taper (10a) and a female type luer taper (10b) are formed in the from end portion and rear end portion of connector (10), respectively.

4. The guide wire assembly according to claim 1, characterized in that the guide wire (11) is formed of a metal wire coated with a synthetic resin layer.

5. The guide wire assembly according to claim 4, characterized in that the metal wire (11) coated with a synthetic resin layer is further coated with a water-soluble high molecular weight material.

6. The guide wire assembly according to claim 1, characterized in that the proximal end of the guide wire (11) is bent at nearly the right angles and the bent proximal end is embedded in the inner wall of the hollow portion of the connector (10).

7. The guide wire assembly according to claim 1, characterized in that the proximal end (11a) of the guide wire (11) is bent in nearly T-shape and the tip portions of T-shaped bent portion are embedded in the inner wall of the hollow portion (10c) of the connector (10).

8. The guide wire assembly according to claim 1, characterized in that the guide wire (11) is coated with an X-ray impermeable substance.

## Claims

1. A guide wire assembly for medical instrument, comprising a hollow connector (10) open at both ends and capable of connection at the rear

F I G. 1

F I G. 2

F I G. 3

F I G. 4

F I G. 5

F I G. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 213 748  (ADVANCED CARDIOVASCULAR SYSTEMS)<br>* Figure 1a; column 4, lines 5-14 *<br>--- | 1-8 | A 61 M  25/01 |
| A | DE-C-3 447 642  (CRAMER)<br>* Figure 1; column 7, lines 50-62 *<br>--- | 1-8 | |
| A | DE-A-2 929 562  (OLYMPUS OPTICAL CO.)<br>* Figure 12 *<br>----- | 1-8 | |

TECHNICAL FIELDS
SEARCHED (Int. Cl.5)

A 61 M

The present search report has been drawn up for all claims

| Place of search | Date of completion of the search | Examiner |
|---|---|---|
| THE HAGUE | 09-03-1990 | MIR Y GUILLEN V. |

EPO FORM 1503 03.82 (P0401)